# EUROPEAN PATENT APPLICATION

(11) **EP 3 269 327 A1**
(43) Date of publication of application: **17.01.2018**
(21) Application number: 16882836.6
(22) Date of filing: 05.01.2016
(51) Int. Cl.: A61F 2/00, A61F 2/14, A61F 9/00, A61F 9/008

(54) **ARRANGEMENT OF INTRASTROMAL SEGMENTS**

(71) Applicant: Albertazzi, Roberto Gustavo, Quilmes, Buenos Aires (AR)
(72) Inventor: Albertazzi, Roberto Gustavo, Quilmes, Buenos Aires (AR)
(74) Representative: CH Kilger Anwaltspartnerschaft mbB
(86) International application number: PCT/CL2016/050001
(87) International publication number: WO 2017/117689

(57) **Abstract**

A disposition of intrastromal links to be implemented in the ophthalmological field, wherein the disposition comprises a plurality of links interconnected to each other, each of the links with a specific geometrical configuration, wherein such geometrical configuration varies according to the ophthalmological condition to be treated; such interconnection among such links is made in a pivoting and flexible manner. Segments are dynamic and allow its adaptation to the tunnel made during the surgical intervention, and they are injectable through a limbal parallel incision away from the tunnels, which can only be performed with Femtosecond Laser technology, where they are placed to avoid the extrusion of segments through the wound. Segments or links and their union allow to intercalate links of different tilt profile, designs and even bases.

## Description

### FIELD OF THE INVENTION

This invention refers to a disposition of intrastromal segments to be implemented in the ophthalmology field, preferably.

### BACKGROUND OF THE INVENTION

Intracorneal rings are two methacrylate arcs used in ophthalmology as a surgical alternative to avoid corneal transplantation as a treatment for corneal ectasias. Once implanted, they regularize such surface and try to stop the pathology from progressing. They partially or fully correct the astigmatism due to the keratoconous disease, improving visual sharpness.

The surgeon places them faced, in ring shape, within the cornea, at its peripheral area, guided through a pre-dissected channel with a specific dissector channeling 4. Most frequent interventions include patients affected by the keratoconous, pellucid marginal degeneration and irregularities in the cornea, which appear after a laser surgery.

Intracorneal segments are used since 1978 for different purposes and from different materials, as dissected corneal stroma, polysulfone and different types of acrylics.

Different diameters (8,5, 6 and 7 mm), profiles (flat with 0° tilt and tapered with 17° and 34° tilt) and designs (Triangular, Trapezoidal, fusiform; Hexagonal) bases (600 microns, 800 microns, 900 microns and even 1330 microns) were used to obtain different effects in the cornea where they are inserted.

Over time, precise technical details were revealed, which showed reduced complications and allow them to earn a space in the treatment of low levels of myopia and corneal ectasias. Tolerance of acrylic material within the deep corneal stroma revealed positive results, improving corneal curvature and visual sharpness.

All these implants are rigid, static and always implanted in the tissue with manual, surgical techniques, which needed a precise cut perpendicular to the stroma to make a 360º tunnel from there in two semi-circles, either in a manual or automated manner, assisted by perilimbal suction rings. The advent of Femtosecond Lasers to the ophthalmological surgery makes us repeat this exact same technique by using the precision the laser technology offers, not changing the way we operate.

### OBJECT OF THE INVENTION

Therefore, the object of this invention is to provide a disposition of intrastromal links connected to each other to be used in the ophthalmological field, wherein such segments are dynamic and allow its adaptation to the tunnel made during the surgical intervention, and they are injectable through a limbal parallel incision away from the tunnels, which can only be performed with Femtosecond Laser technology, where they are placed to avoid the extrusion of segments through the wound.

Another object of this invention is to provide a disposition of intrastromal links to be implemented in the ophthalmological field, wherein the links and their union allow to intercalate links of different tilt profile, designs and even bases.

Therefore, the object of this invention is to provide a disposition of intrastromal links to be implemented in the ophthalmological field, wherein the disposition comprises a plurality of links interconnected to each other, each of the links with a specific geometrical configuration, wherein such geometrical configuration varies according to the ophthalmological condition to be treated; such interconnection among such links is made in a pivoting and flexible manner.

### DESCRIPTION OF FIGURES

For more clarity and understanding of the object of this invention, it has been illustrated in several figures, which represent the invention in one of the preferred forms of embodiment, as an example, wherein:
Figure 1 is a perspective view of the disposition object of this invention; and
Figure 2 is a perspective view of the disposition of figure 1;
Figure 3 is a plan view from above the disposition of figure 1; and
Figures 4a and 4b are perspective views of the links or segments comprising the disposition of figure 1.

### DETAILED DESCRIPTION OF THE EXAMPLE OF THE EMBODIMENT

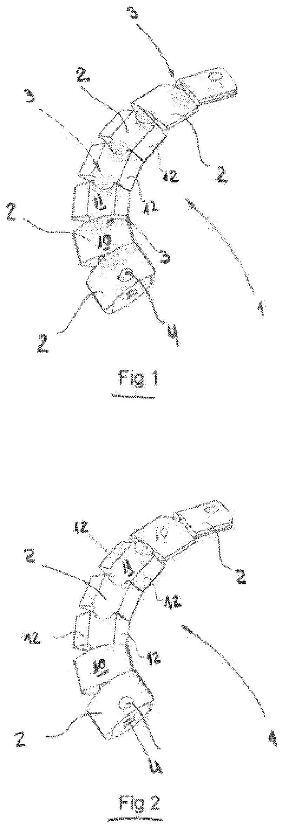

By referring to the figures, it can be observed the disposition of intracorneal segments object of this invention, marked with the general reference number 1. In this particular embodiment, such disposition 1 comprises a plurality of links or segments 2, each of them featuring a specific geometrical configuration, which varies according to the ophthalmological condition to be treated. As described in figures 1-3, segments 2 are interconnected to each other through interconnection means 3. These interconnection means are defined by a female connector 4 located in the proximal end 5 of the link 2, and by a male connector 6, which is located in the distal end 7 of such link 3. It is important to note that the interconnection among links 2 is made in a flexible manner. This allows the links to move laterally, avoiding anteroposterior movement.

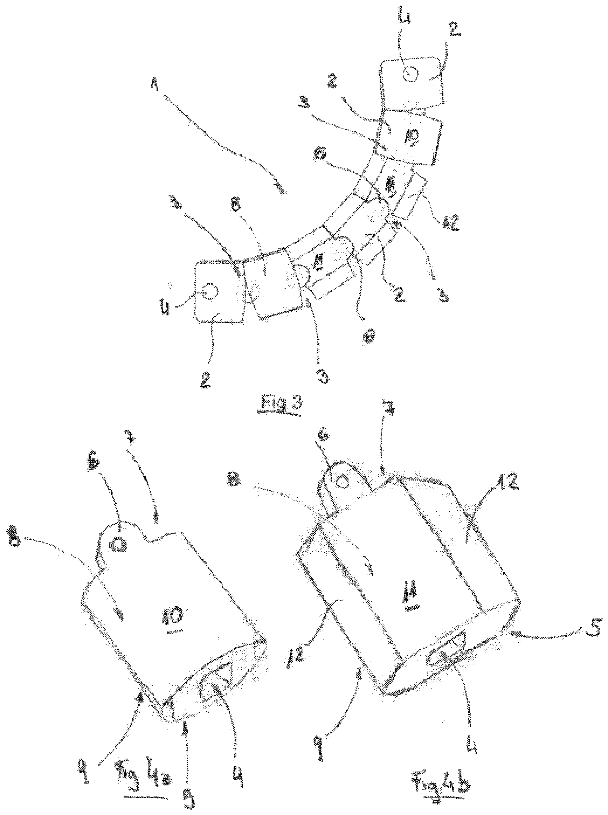

With regard to links, and preferably the ones illustrated in figures 4a and 4b, it can be observed that they have a substantially rectangular formation, with a top surface 8 and a bottom surface 9. For the link illustrated in figure 4a, the top surface 8 has a substantially convex curvature 10. While the bottom surface 8 of the link in the figure 4b has a medium flat portion 11 and perimeter edges 12 with a chamfer in each edge 12. It should be noted that, in this particular embodiment, such chamfer in the edges 12 of the link 2 has a tilt of 10º-40º, preferably 17º-35º.

Intracorneal segments may have different tilt, design and base profiles. Intracorneal segments are small implants of rigid medical grade acrylic (PMMA), which pass tolerance and compatibility tests regarding the corneal tissue in which they are implanted. Since they are rigid, when implanted in a viscoelastic structure with prolate aspheric shape like the cornea, they distort this structure in a different manner, according to the implants. For instance, flat implants produce an effect, while tapered with tilt from 17º to 34º will produce another effect. Segments act through different mechanisms; one of them are the changes of their thickness, wherein the greater the thickness is the greater the effect, and vice versa. It also changes its effect according to the diameter, wherein the smaller the diameter is the greater the effect, and vice versa.

Another mechanism through which they act is the so-called arc reduction, in which all of them increment the tension of collagen fibers of anterior stroma producing flattening. The flatter the angulation of the segment is with respect to the horizontal axis, the greater the corneal flattening effect will be, since it applies a force against the physiological angulation of the cornea.

Now, the design of links or segments is rigid with respect to superoinferior torsion, which is a necessary feature for a durable corneal effect. However, its rigid side is necessary for the insertion. As it includes different modules, each of them has different tilts, designs and even different bases. Therefore, the volume and diameter will also change.

Due to the FEMTO LASER technology, these segments are implanted thanks to their injectable design from an incision away from the area they produce an effect to, such as the limbal, which heals much easier because it is next to the conjunctival stem cells and near the blood vessels. For this reason, this area is very physiological and the zone for all the cataract surgeries.

The disposition object of this invention applies to the treatment of keratoconous. The keratoconous is an inherited congenital disease characterized by a non-inflammatory, progressive corneal deformation: it has 4 progressive states from a mild, imperceptible corneal deformation to the lost of corneal structure and function needed for a corneal insert, which still cannot be cured nowadays. These types of deformations are completely asymmetrical, central, paracentral and peripheral of which there are many descriptions and described patterns. Rigid segments currently used are to stabilize these deformations and even to reduce them to recover the vision lost due to deformations caused by the disease. These injectable segments have an incredible potential, since they can be made to measure for each deformation and each cornea, and even be customized with maps of corneal curvature and thickness. Possibilities are almost endless.

The design of the segments comprised in the disposition allows a simple implantation in a specific area, where the Femto Laser makes the tunnel and creates the channel where they will be placed, and once in that area, the only force required for its effect is resistance to torsion by the tissue.

It is worth noticing that the segments are made of rigid materials like the ones used nowadays, i.e., polymethylmethacrylate. Note that they can be made of porous materials (or with micro-tunnels), allowing the insertion of cell culture in their interior.

## Claims

1. A disposition of intrastromal links to be implemented in the ophthalmological field, wherein the disposition comprises a plurality of links interconnected to each other, each of the links with a specific geometrical configuration, wherein such geometrical configuration varies according to the ophthalmological condition to be treated; such interconnection among such links is made in a pivoting and flexible manner.

2. The disposition according to the claim 1, wherein such interconnection allows the lateral movement of the links, avoiding their anteroposterior movement.

3. The disposition according to the claim 1, wherein at least one of such links comprises a substantially rectangular formation, with a top surface and a bottom surface.

4. The disposition according to the claim 3, wherein such top surface comprises a substantially convex curvature.

5. The disposition according to the claim 3, wherein such top surface comprises a medium flat portion and perimeter edges with chamfer.

6. The disposition according to the claim 5, wherein such chamfer comprises a tilt of 10º to 40º and preferable of 17º to 35º.

7. The disposition according to the claim 1, wherein each of the links comprises an interconnection media defined by a female connector and a male connector.

8. The disposition according to the claim 7, wherein such female connector is located at the distal end of the link.

9. The disposition according to the claim 7, wherein such male connector is located at the proximal end of the link.

10. The disposition according to any of the claims above, wherein such links are made of a material that allows stiffness against supero-inferior torsion, without side stiffness.

11. The disposition according to any of the claims above, wherein such links are made of a porous material defining conducts for the insertion of cell culture in their interior.
